## Europäisches Patentamt
## European Patent Office
## Office européen des brevets

(11) Veröffentlichungsnummer: **0 209 052**
**B1**

(12) **EUROPÄISCHE PATENTSCHRIFT**

(45) Veröffentlichungstag der Patentschrift:
06.09.89

(51) Int. Cl.⁴: **A 61 M 5/14**

(21) Anmeldenummer: **86109295.5**

(22) Anmeldetag: **08.07.86**

(54) **Vorrichtung zur Infusion und Entnahme von Proben aus Blut.**

(30) Priorität: **11.07.85 DE 3524824**

(43) Veröffentlichungstag der Anmeldung:
**21.01.87 Patentblatt 87/4**

(45) Bekanntmachung des Hinweises auf die Patenterteilung:
**06.09.89 Patentblatt 89/36**

(84) Benannte Vertragsstaaten:
**DE FR GB IT**

(56) Entgegenhaltungen:
**WO-A-84/04033**
**DE-A- 2 644 062**
**US-A- 3 946 731**
**US-A- 4 077 395**
**US-A- 4 258 717**

(73) Patentinhaber: **Fresenius AG, Gluckensteinweg 5,
D-6380 Bad Homburg v.d.H. (DE)**

(72) Erfinder: **Polaschegg, Hans-Dietrich, Dr.,
Grünwiesenweg 9, D-6370 Oberursel 4 (DE)**

(74) Vertreter: **Görtz, Dr. Fuchs, Dr. Luderschmidt
Patentanwälte, Sonnenberger
Strasse 100 Postfach 26 26, D-6200 Wiesbaden (DE)**

ACTORUM AG

## Beschreibung

Die Erfindung betrifft eine Vorrichtung zur Infusion und Entnahme von Proben aus Blut und anderen Körperflüssigkeiten gemäß dem Oberbegriff des Anspruchs 1 bzw. 3 sowie ein Verfahren zum Betrieb dieser Vorrichtung gemäß Anspruch 2 bzw. 4.

Im Rahmen der Intensivmedizin ist die Bestimmung von Blutwerten, wie z.B. des Gesamteiweißes oder der Elektrolyte, von besonderer Bedeutung. Wird beispielsweise während einer Intensivbehandlung eine Infusionstherapie mittels Kalium vorgenommen, ist eine quasi-kontinuierliche Überwachung der Blutkaliumwerte erforderlich, da eine Über- oder Unterdosierung schwerwiegende Folgen haben kann.

Bei einer derartigen Therapie wird zur Überwachung der Blutwerte üblicherweise dem Patienten über einen separaten zusätzlichen Blutanschluß, wie beispielsweise einen Katheter, das für die Untersuchung erforderliche Blut entnommen. Hierbei ist jedoch nachteilig, daß der Patient durch diesen weiteren Blutentnahmekatheter zusätzlich belastet wird.

Aus der DE-A-3 313 074 ist eine solche Vorrichtung bekannt, mit der Blut und andere Körperflüssigkeiten aus einem Patienten entfernt und einem Probennahmegerät zugeführt werden können. Nach der Entnahme der gerinnbaren Körperflüssigkeiten wird das Gerät intermittierend mit einer Spüllösung gespült, worauf wiederum der Entnahmebetrieb einsetzt. Die bekannte Vorrichtung wird jedoch nicht zur Infusion einer Flüssigkeit eingesetzt und ist vielmehr eine Blutentnahmevorrichtung, die zusätzlich zu einer Infusionsvorrichtung eingesetzt wird, was zu den vorstehend erwähnten Nachteilen führt.

Aus der DE-A-2 644 062 und der DE-A-3 335 744 sind Single-Needle-Hämodialysevorrichtungen bekannt, bei denen Blut in einem extrakorporalen Kreislauf durch einen Dialysator geführt und dort von den Stoffwechselprodukten befreit wird. Eine derartige Hämodialysemaschine ist jedoch weder eine Infusionsvorrichtung noch eine Probenahmeeinrichtung, sondern vielmehr eine Blutreinigungsmaschine, die dem Zwecke der Reinigung und nicht der Infusion oder der Probennahme dient.

Der Erfindung liegt daher die Aufgabe zugrunde, die Vorrichtung gemäß dem Oberbegriff des Anspruchs 1 bzw. 3 so fortzubilden, daß mit ihr wechselweise eine Infusion oder eine Probennahme durchgeführt werden kann, sowie ein Verfahren zum Betrieb dieser Vorrichtung zur Verfügung zu stellen.

Diese Aufgabe wird durch die kennzeichnenden Merkmale des Anspruchs 1 bzw. 3, soweit die Vorrichtung betroffen ist, und die kennzeichnenden Merkmale des Anspruchs 2 bzw. 4 gelöst, soweit das Verfahren betroffen ist.

Erfindungsgemäß wird nunmehr eine Vorrichtung zur Verfügung gestellt, mit der über einen einzigen Blutanschluß sowohl eine Infusion als auch eine Entnahme von Blut oder anderen Körperflüssigkeiten durchgeführt werden kann. Hierdurch wird die ohnehin große Belastung des Patienten auf ein Minimum reduziert, wobei zugleich eine Überwachung der Blutentnahme des Patienten während der Infusion sichergestellt wird.

Während der Therapie kann die erfindungsgemäße Vorrichtung mittels der Steuereinrichtung derart alternierend entsprechend einem vorbestimmten Programm, also in vorbestimmten Zeitabständen, vom Infusionsbetrieb in den Blutentnahmebetrieb und umgekehrt geschaltet werden. Erfindungsgemäß können die im Leitungssystem angeordneten Absperrorgane in Zeitabständen vom Infusionsbetrieb in den Blutentnahmebetrieb und umgekehrt geschaltet werden. Dabei werden die im Leitungssystem angeordneten Absperrorgane ebenso unterschiedlich wie eine vorzugsweise als Hubkolbenpumpe ausgebildete Pumpe betätigt, die wechselweise in den Expansionsbetrieb (Füllbetrieb) beziehungsweise den Kompressionsbetrieb (Entleerungsbetrieb) geschaltet wird. Diese Pumpe wird gleichermaßen nacheinander sowohl mit Infusionslösung als auch mit Blut oder anderen Körperflüssigkeiten beaufschlagt, wobei sichergestellt wird, daß sämtliche Flüssigkeiten zum Patienten zurückgefördert werden, also sämtliche Flüssigkeiten das extrakorporale System nur während einer kurzen Zeitspanne durchlaufen. Insofern besteht also nicht die Gefahr, daß durch Gerinnung von Blut oder anderen Körperflüssigkeiten das extrakorporale Leitungssystem verstopft wird. Dies wird im übrigen vorzugsweise dadurch verhindert, daß die Infusionslösung zugleich als Spüllösung dient, so daß bei dem auf den Blutentnahmebetrieb folgenden Infusionsbetrieb der erfindungsgemäßen Vorrichtung das im extrakorporalen Kreislauf befindliche Blut durch die Infusionslösung verdrängt wird, wobei zugleich eine Freispülung des gesamten extrakorporalen Systems erfolgt.

Gemäß einer ersten bevorzugten Ausführungsform wird der extrakorporale Kreislauf in einen geschlossenen Kreis geführt, der lediglich einen zum Patienten führenden Anschluß aufweist. Desweiteren ist dieser Kreislauf mit wenigstens einem Infusionslösungsbehälter verbunden, wobei zusätzlich in den Kreislauf eine Probennahmeeinrichtung eingeschaltet ist, an der entweder Blutproben abgenommen oder aber die Blutparameter gemessen werden können.

Als derartige Probennahmeeinrichtung eignen sich beispielsweise Durchflußsensoren, mit denen bestimmte Blutparameter, beispielsweise der Natrium-, Kalium- oder Kalziumgehalt des Bluts, der Hämotokritwert, die Leitfähigkeit des Bluts und dgl. gemessen werden können.

Andererseits kann jedoch aber auch ein Membranfilter als Probennahmeeinrichtung eingesetzt werden, an dem beispielsweise Blutplasma entnommen werden kann, in dem die oben erwähnten Parameter in einem weiteren Gerät bestimmt werden können. Zusätzlich können noch weitere Parameter, beispielsweise Eiweißfraktionen und dgl., wobei das Plasma derartige Eiweiße aufweist, bestimmt werden.

Schließlich kann als Blutentnahmeeinrichtung natürlich auch eine Blutentnahmestelle vorgesehen sein, an der beispielsweise mittels einer Spritze Blut entnommen wird, das in einem weiteren Analysator analysiert wird.

Gemäß dieser ersten Ausführungsform kann also sowohl die Infusionslösung als auch das Blut im Kreis geführt werden, wobei lediglich im Bereich des Anschlusses des extrakorporalen Systems mit dem Katheter ein rezirkulierender Bereich zu finden ist.

Gemäß einer bevorzugten zweiten Ausführungsform wird die eine Seite eines extrakorporalen Schlauchsystems mit dem Katheter verbunden, während die andere Seite des Schlauchs mit der Pumpkammer verbunden wird. In diesem Schlauch ist die Probennahmeeinrichtung eingeschaltet, so daß Blut beim Aspirieren durch die Pumpe zwangsläufig durch die Probennahmestation geführt wird. Nach dem Füllen der Pumpe wird das Blut auf dem gleichen Weg wieder zurück in den Patienten gepumpt. Anschließend wird das gesamte extrakorporale System wiederum mit der Infusionslösung gefüllt, wobei zugleich eine Reinigung des Systems erfolgt.

Erfindungsgemäß ist es dabei wichtig, daß die im extrakorporalen Schlauchsystem befindlichen Flüssigkeiten nicht den Probennahmevorgang, insbesondere die unmittelbare Messung der Blutparamter stören bzw. verfälschen. Insofern muß also Sorge dafür getragen werden, daß die Pumpe bei dem Blutentnahmebetrieb sämtliche im extrakorporalen Schlauchsystem verbliebenen Infusionslösungsreste und zusätzlich die mit diesen Resten vermischten Blutbestandteile aufnimmt, so daß in der Probennahmeeinrichtung nach der vollständigen Füllung der Pumpe eine von Verunreinigungen freie Blutprobe zur Messung gelangen kann.

Vorteilhafterweise entspricht dabei das Aufnahmevermögen der Pumpe wenigstens etwa dem Füllvolumen des extrakorporalen Systems, das von der Spitze des Katheters bis zur Blutpumpe reicht. Hierdurch wird sichergestellt, daß sämtliche in dem extrakorporalen System verbliebenen Infusionsflüssigkeitsreste von der Pumpe aufgenommen werden können und zusätzlich ein bestimmter mit Infusionslösung verunreinigter Blutrest teilweise von der Pumpe aufgenommen wird und teilweise in dem Leitungsrest verbleibt, der stromab der Probennahmeeinrichtung zur Pumpe gemäß der zweiten Ausführungsform führt.

Die Unteransprüche haben vorteilhafte Weiterbildungen der Erfindung zum Inhalt.

Weitere Einzelheiten, Merkmale und Vorteile der Erfindung ergeben sich aus nachfolgender Beschreibung zweier Ausführungsbeispiele anhand der Zeichnung.

Es zeigt:

Fig. 1 eine schematische Darstellung einer ersten Ausführungsform der erfindungsgemäßen Vorrichtung und

Fig. 2 eine der Fig. 1 entsprechende Darstellung einer zweiten Ausführungsform der erfindungsgemäßen Vorrichtung.

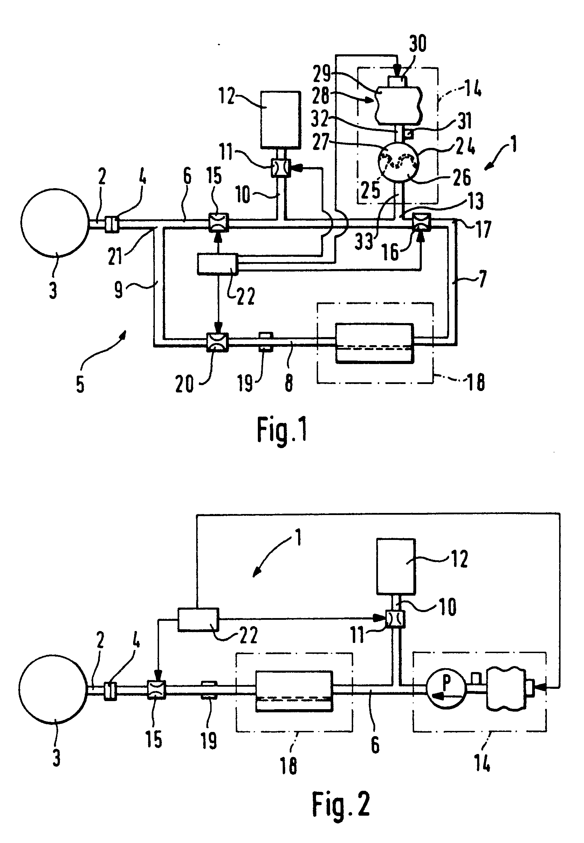

Eine in Fig. 1 dargestellte erste Ausführungsform der erfindungsgemäßen Vorrichtung 1 zur Probennahme und Infusion weist einen Blutanschluß 2 auf, der in Form eines üblichen Katheters ausgebildet sein kann. Der Blutanschluß 2 ist in den in Fig. 1 mittels des Kreises 3 symbolisierten Patienten eingesetzt. An den Blutanschluß 2 ist über einen Konnektor 4 ein Leitungssystem 5 angeschlossen, das im Beispielsfalle aus vier Leitungsabschnitten 6, 7, 8 und 9 besteht. An den Leitungsabschnitt 6 ist eine erste Zweigleitung 10 angeschlossen, in der ein Absperrorgan 11 angeordnet ist, und die mit einem Infusionslösungsbehälter 12 in Verbindung steht. Weiterhin ist an den ersten Leitungsabschnitt 6 eine zweite Zweigleitung 13 angeschlossen, die mit einer Pumpe 14 in Verbindung steht, die vorzugsweise als pneumatisch angetriebene Pumpe ausgebildet ist.

Ferner ist in dem Leitungsabschnitt 6 zwischen dem Blutanschluß 2 und der Zweigleitung 10 ein Absperrorgan 15 abgeordnet. Ein weiteres Absperrorgan 16 ist in dem Leitungsabschnitt 6 zwischen der Pumpe 14 und einem Verzweigungspunkt 17 in dem Leitungsabschnitt 6 und dem Leitungsabschnitt 7 angeordnet.

In dem Leitungsabschnitt 8 ist eine Meßeinrichtung 18 vorgesehen, mittels der die zu untersuchenden Blutparameter bestimmt werden können. Ferner weist der Leitungsabschnitt 8 einen Luftdetektor 19 und ein weiteres Absperrorgan 20 auf, das zwischen der Meßeinrichtung 18 und dem Zurückführungspunkt 21 des Leitungsabschnittes 9 in dem Leitungsabschnitt 6 angeordnet ist.

Der Luftdetektor 19 kann auch im Leitungsabschnitt 6 angeordnet sein, um die Luftinfusion in beiden Betriebsarten als Infusionspumpe zu verhindern.

Ferner ist eine schematisch vereinfacht dargestellte Steuereinrichtung 22 vorgesehen, die zur Steuerung der Absperrorgane 11, 15, 16 und 20 dient.

Im folgenden wird das Wirkungsprinzip der erfindungsgemäßen Vorrichtung 1 beschrieben.

Durch die vorzugsweise pneumatisch angetriebene Pumpe 14 kann durch die geeignete Schaltung der Absperrorgane 11, 15, 16 und 20 dem Patienten wahlweise entweder Blut entnommen und über die Meß- bzw. Probennahmeeinrichtung 18 zurückinfundiert oder Infusionslösung aus dem Infusionslösungsbehälter 12 dem Patienten infundiert werden. Hierzu werden für die Probennahme die Absperrorgane 11, 16 und 20 geschlossen, während das Absperrorgan 15 geöffnet wird. In diesem Zustand kann die pneumatische Pumpe 14 Blut aus dem Patienten ansaugen. Ist der Ansaugvorgang beendet, wird das Absperrorgan 15 geschlossen und die Absperrorgane 16 und 20 werden geöffnet. Hierauf komprimiert die Pumpe 14 und fördert das angesaugte Blut über die Probennahmeeinrichtung 18 zurück in den Patienten. Hierbei verhindert der Luftdetektor 19, der mittels üblicher Sicherheitsschaltungen gesteuert wird, daß Luft in den Patienten gefördert wird. Ist die Probennahme und die Messung abgeschlossen, kann die erfindungsgemäße Vorrichtung 1 auf Infusion umgeschaltet werden.

Dazu wird das Absperrorgan 15 zunächst geschlossen, während zum Ansaugen von Infusionslösung das Absperrorgan 11 geöffnet wird. Hiernach wird zum Infundieren das Absperrorgan 11 wieder geschlossen, wohingegen die Absperrorgane 16 und 20 geöffnet werden. In diesem Zustand der Absperrorgane wird die von der Pumpe 14 angesaugte Infusionslösung über die Leitungsabschnitte 7, 8, 9 und den vom Konnektor 4 zum Zurückführungspunkt 21 führenden Teil des Leitungsabschnittes 6 dem Patienten zugeführt.

Alternativ zu dieser zuvor beschriebenen Schaltung der Absperrorgane ist es jedoch auch möglich, nach dem Ansaugen der Infusionslösung das Absperrorgan 16 zu schließen und das Absperrorgan 15 zu öffnen, wonach die Infusionslösung über den Leitungsabschnitt 6 direkt zum Patienten gefördert werden kann.

Für die Funktion der Vorrichtung 1 sind folgende Randbedingungen einzuhalten:

Der nicht ständig durchspülte Bereich des Leitungsabschnittes 6 zwischen dem Punkt 21 und dem Absperrorgan 15 soll so kurz wie möglich gehalten werden, damit verhindert werden kann, daß darin Blut stehen bleibt und gerinnt. Das Pumpvolumen der pneumatischen Pumpe 14 ist so groß auszulegen, daß Blut bzw. Flüssigkeit nicht nur hin und her bewegt werden, sondern tatsächlich dem Patienten entnommen und wieder zurückgeführt werden kann. Das bedeutet, daß das Pumpvolumen der Pumpe 14 deutlich größer sein muß als das Füllvolumen des Katheters bis zum Punkt 21. Diesbezüglich ergibt die zuvor beschriebene zweite Möglichkeit zur Infusion lediglich über den Leitungsabschnitt 6 den Vorteil, daß auf diese Art und Weise in diesem Bereich der Vorrichtung befindliches Blut mittels der Infusionslösung dem Patienten zurückgeführt werden kann. Werden beide zuvor beschriebenen Infusionsmöglichkeiten nacheinander durchgeführt, ist es möglich, auf diese Weise die gesamte Vorrichtung von Blutresten zu befreien.

Gem. Fig. 2 ist eine konstruktiv vereinfachte Ausführungsform der erfindungsgemäßen Vorrichtung 1 dargestellt. Bei der nachfolgenden Beschreibung sind gleiche Teile mit gleichen Bezugszeichen wie in Fig. 1 bezeichnet.

Wie aus der Darstellung ersichtlich ist, weist die gem. Fig. 2 dargestellte Ausführungsform der Vorrichtung 1 lediglich zwei Absperrorgane 15 und 11 auf, die in dem Leitungsabschnitt 6 bzw. der hier einzigen Zweigleitung 10 angeordnet sind. Hinsichtlich ihrer Funktion unterscheidet sich die zweite Ausführungsform von der ersten dadurch, daß im Probennahmebetrieb Blut nicht umgepumpt, sondern lediglich hin und zurück gepumpt wird.

Zur Probenahme wird die Pumpe 14 zunächst komprimiert, das Absperrorgan 11 geschlossen und das Absperrorgan 15 geöffnet. Daraufhin kann Blut angesaugt werden, wobei zunächst Infusionslösung aus dem Blutanschluß 2 zurückgesaugt wird, wonach Blut folgt. In der oberen Totpunktlage der Pumpe 14 kann dann eine Probe entnommen werden, wonach anschließend das Blut zurückinfundiert wird. Daraufhin wird die Pumpe 14 als Infusionspumpe betrieben, wobei beim Ansaugvorgang das Absperrorgan 15 geschlossen wird, während das Absperrorgan 10 geöffnet ist. Beim Fördern von Infusionslösung hingegen ist das Förderorgan 11 geschlossen und das Förderorgan 15 geöffnet. Zur Funktionsfähigkeit dieser Ausführungsform muß vorausgesetzt werden, daß das Pumpvolumen der Pumpe 14 wesentlich größer ist als das Volumen der Leitung von der Pumpe 14 bis zum Ende des Blutanschlusses 2.

Wie aus Fig. 1 und 2 ersichtlich ist, dient die Pumpe zum Fördern der Infusionslösung oder des Bluts bzw. der Körperflüssigkeiten. Vorteilhafterweise ist diese Pumpe 14 als pneumatische Pumpe ausgebildet und weist — wie in Fig. 1 und 2 gezeigt ist — eine Pumpkammer (24) auf, die durch eine flexible Membran (25) in einen ersten Pumpkammerteil (26) und einen zweiten Pumpkammerteil (27) geteilt ist.

Beide erste und zweite Pumpkammerbereiche bilden zusammen das Innenvolumen $V_1$ der Pumpkammer (24).

Die flexible Membran (25) kann sich in Abhängigkeit von der Betriebsphase der Pumpe, der Kompression — oder der Expansionsphase — in entgegengesetzten Richtungen soweit bewegen, bis sie sich völlig an die Innenwand der Pumpkammer angelegt hat. Dies ist beispielsweise in der Ausführungsform gemäß Fig. 2 im Expansionszustand der Pumpe 14 gezeigt.

Wie aus der Zeichnung ersichtlich ist, ist die Pumpkammer (24), insbesondere der zweite Pumpkammerteil (27) mit dem Antriebsteil (28), das aus einem pneumatisch betriebenen Balg (29), einem Antriebsmotor (30) und einer Ventileinheit (31) besteht, über eine Verbindungsleitung (32) verbunden.

Demgegenüber ist der erste Pumpkammerteil (26) mit dem Leitungsabschnitt (6) über eine weitere Verbindungsleitung (33) verbunden. Hinzufügen ist, daß diese Zwischenleitung (33) auch entfallen kann, d.h. der erste Pumpkammerteil (26) unmittelbar in die Leitung (6) eingeschaltet sein kann.

Der Balg (29) kann von dem Antriebsmotor (30) entweder komprimiert oder aber expandiert werden, wobei die Umschaltung der Pumpe (14) von der einen in die andere Phase jeweils an den unteren und oberen Totpunkten der Pumpe erfolgt. Mit dieser Umschaltung erfolgt synchron die jeweilige Umschaltung der Absperrorgane (11, 15, 16 und 20) in die andere Betriebsphase durch die Steuereinheit (22).

Schließlich sei noch erwähnt, daß die Ventileinheit (31) zur Belüftung der Verbindungsleitung (32) und damit des Balgs (29) nach einem bestimmten Programm eingesetzt werden kann, wie es beispielsweise in der DE-OS 3 205 449 beschrieben ist, die ebenfalls eine derartige Balgpumpe beschreibt. Zu Offenbarungszwecken wird ausdrücklich auf diese Offenlegungsschrift bezug genommen.

Das Innenvolumen $V_1$ der Pumpkammer (24) ist — wie vorstehend erwähnt — wesentlich größer als die Innenvolumina der Leitungen einschließlich des Katheters, um ein Hin- und Herpumpen der Infusionslösung bzw. vom Blut in dem Leitungssystem zu unterbinden. Vorzugsweise ist das Volumen der Pumpkammer wenigstens doppelt so groß wie das gesamte Innenvolumen des Leitungssystems.

Wie bereits eingangs erwähnt, kann als Probenahmeeinheit (18) ein elektrochemisches Sensorsystem eingesetzt werden, mit dem daß vorteilhafterweise selektiv bestimmte Blutparameter, beispielsweise das Natrium-, Kalium- oder das Kalziumion detektiert werden können. Eine derartige Anordnung ist beispielsweise in der DE-A-3 416 956 beschrieben, auf deren Offenbarung ausdrücklich Bezug genommen wird.

Vorteilhaft beim Einsatz von elektrochemischen Sensoren ist dabei die Tatsache, daß die zu infundierende Lösung infolge ihrer gleichbleibenden Zusammensetzung als Spüllösung und Eichstandart für den elektrochemischen Sensor zwischen den jeweiligen Bestimmungen der Blutparameter eingesetzt werden kann, so daß jeweils eine genau kalibrierte Meßordnung für die durchzuführende Messung zur Verfügung steht.

Ergänzend sei noch darauf hingewiesen, daß die beschriebene Vorrichtung nicht unbedingt simultan als Infusionspumpe benutzt werden muß. Um jedoch eine Gerinnung des Blutes zu vermeiden, ist es vorteilhaft, nach der Messung die Probennahme und Meßeinrichtung mit einer Infusionslösung zumindest freizuspülen. Wird dabei ein Teil dieser Lösung dem Patienten mit infundiert, muß dieser Teil in die Flüssigkeits- und Stoffbilanz eingerechnet werden.

**Patentansprüche**

1. Vorrichtung zur Probennahme von Blut oder anderen Körperflüssigkeiten mit einem Anschluß (2) an ein Körpergefäß (3), von dem eine erste Leitung (6 - 9) abgeht, die eine Meß- bzw. Probenentnahmeeinrichtung (18) und eine Pumpe (14) aufweist, einem mit der ersten Leitung (6 - 9) über eine zweite Leitung (10) verbundenen, eine physiologisch verträgliche wässerige Lösung enthaltenden Behälter (12), einem ersten in die zweite Leitung (10) eingeschalteten Absperrorgan (11) und einer Steuereinrichtung (22) zur Aktivierung des ersten Absperrorgans (11) und der Pumpe (14), dadurch gekennzeichnet, daß das hintere Ende der ersten Leitung (6 - 9) unter Bildung eines geschlossenen Kreislaufs zum vorderen Ende der ersten Leitung zurückgeführt ist, die Meß- bzw. Probenentnahmeeinrichtung (18) in die erste Leitung (6 - 9) unter Aufspaltung der ersten Leitung in Zuleitungsabschnitte (6, 7) und Ableitungsabschnitte (8, 9) eingeschaltet ist, in den ersten Zuleitungsabschnitt (6) zwischen dem Verbindungspunkt (21) mit dem zweiten Ableitungsabschnitt (9) und dem Verbindungspunkt mit der zweiten Leitung (10) ein zweites Absperrorgan (15) eingeschaltet ist, stromab des zweiten Absperrorgans (15) der erste Zuleitungsabschnitt (6) mit der Pumpe (14) versehen ist und stromab der Pumpe (14) ein drittes Absperrorgan (16) in den ersten Zuleitungsabschnitt (6) eingeschaltet ist, in den ersten Ableitungsabschnitt (8) ein viertes Absperrorgan (20) eingeschaltet ist, das Füllvolumen der Pumpe (14) deutlich größer als das Füllvolumen von dem Anschluß (2) bis zum Verbindungspunkt (21) mit dem ersten Zuleitungsabschnitt (6) und dem zweiten Ableitungsabschnitt (9) ist, die physiologisch verträgliche wässerige Lösung eine Infusionslösung ist und die Steuervorrichtung (22) die Absperrorgane (11, 15, 16, 20) und die Pumpe (14) wechselweise in den Flüssigkeitszuführungsbetrieb und den Blutentnahmebetrieb schaltet.

2. Verfahren zum Betrieb der Vorrichtung nach Anspruch 1, dadurch gekennzeichnet, daß die Steuervorrichtung (22) im Infusionsbetrieb die Pumpe (14) in die Expansionsphase (Fülltakt) schaltet, das erste Absperrorgan (11) öffnet und das zweite und dritte Absperrorgan (15 und 16) schließt und im Entleerungstakt die Pumpe (14) in die Kompressionsphase schaltet, das erste Absperrorgan (15) oder das dritte und vierte Absperrorgan (16, 20) öffnet und daß die Steuervorrichtung (22) im Blutentnahmebetrieb das erste Absperrorgan (11) schließt, das zweite Absperrorgan (15) öffnet, die dritten und vierten Absperrorgane (16, 20) schließt und die Pumpe (14) in die Expansionsphase schaltet und nach dem Füllen der Pumpe (14) die Absperrorgane (15, 16, 20) und die Pumpe (14) in die Kompressionsphase umschaltet.

3. Vorrichtung zur Probenentnahme von Blut oder anderen Körperflüssigkeiten mit einem Anschluß (2) an ein Körpergefäß (3), von dem eine erste Leitung (6) abgeht, die eine Meß- bzw. Probenentnahmeeinrichtung (18) und eine Pumpe (14) aufweist, einem mit der ersten Leitung (6) über eine zweite Leitung (10) verbundenen, eine physiologisch verträgliche wässrige Lösung enthaltenden Behälter (12), einem ersten in die zweite Leitung (10) eingeschalteten Absperrorgan (11) und einer Steuervorrichtung (22) zur Aktivierung des ersten Absperrorgans (11) und der Pumpe (14), dadurch gekennzeichnet, daß die Pumpe (14) mit dem dem Anschluß (2) gegenüberliegenden Ende der ersten Leitung (6) verbunden ist und ein Füllvolumen aufweist, das wesentlich größer ist als das Innenvolumen der ersten Leitung (6), die erste Leitung (6) im Bereich des Blutanschlusses (2) ein zweites Absperrorgan (15) aufweist, die physiologisch verträgliche wässrige Lösung eine Infusionslösung ist und die Steuervorrichtung (22) die Absperrorgane (11, 15) wechselweise in den Flüssigkeitszuführungsbetrieb und den Blutentnahmebetrieb schaltet.

4. Verfahren zum Betrieb der Vorrichtung nach Anspruch 3, dadurch gekennzeichnet, daß die Steuervorrichtung (22) im Infusionsbetrieb die Pumpe (14) in die Expansionsphase (Fülltakt) schaltet, das erste Absperrorgan (11) öffnet und das zweite Absperrorgan (15) schließt und nach dem Füllen die Pumpe (14) in die Kompressionsphase (Entleerungstakt) schaltet, das erste Absperrorgan (11) schließt und das zweite Absperrorgan (15) öffnet und daß die Steuervorrichtung (22) im Blutentnahmebetrieb das erste Absperrorgan (11) schließt, das zweite Absperrorgan (15) öffnet, die Pumpe (14) in die Expansionsphase (Fülltakt) schaltet, wobei das entnommene Blut durch die Probenentnahmeeinrichtung (18) geführt wird, danach die Pumpe (14) in die Kompressionsphase (Entleerungstakt) schaltet und nach Entleerung der Pumpe (14) wieder in den Infusionsbetrieb umschaltet.

5. Vorrichtung nach Anspruch 1 oder 3, dadurch gekennzeichnet, daß ein Luftdetektor (19) in den Leitungsabschnitten (6, 7, 8, 9) angeordnet ist.

6. Vorrichtung nach Anspruch 1 oder 3, dadurch gekennzeichnet, daß die Pumpe (14) eine pneumatisch betriebene Pumpe ist, die zwischen einer Kompressionsphase und einer Expansionsphase pendelt.

7. Vorrichtung nach Anspruch 6, dadurch gekennzeichnet, daß die Pumpe (14) aus einer Pumpkammer (24), die durch eine flexible Membran (25) in einen ersten Pumpkammerteil (26) und einen zweiten Pumpkammerteil (27) geteilt ist, und aus einem pneumatischen Antriebsteil (28) für die Pumpkammer (24) besteht.

8. Vorrichtung nach einem der Ansprüche 1, 3, 5, 6 oder 7, dadurch gekennzeichnet, daß die Probennahmeeinrichtung (18) eine elektrochemische Meßanordnung oder eine Filtereinheit zur Abtrennung von Plasma ist.

## Claims

1. Apparatus for taking of samples of blood and other body fluids comprising a port (2) to a body vessel (3) of which a first line (6 - 9) extends, said line having a sample measuring or respectively taking means (18) and a pump (14), further comprising a container (12) being connected to said first line (6 - 9) via a second line (10) and having a physiologically compatible aqueous solution, further comprising a first shut-off means (11) being disposed in said second line (10) and a control means (22) for activating said first shut-off means and said pump (14), characterized in that said rear end of said first line (6 - 9) is led back to said front end of said first line and forms a closed circuit, that the sample measuring or respectively taking means (18) is connected to said first line (6 - 9) and separates said first line into incoming line segments (6, 7) and outgoing line segments (8, 9), that into said first incoming line (6) between the connection point (21) with the second outgoing line segment (9) and the connection point with the second line (10) a second shut-off means (15) is disposed, that downstream of said second shut-off means (15) said first incoming line segment (6) is provided with said pump (14) and downstream of the pump (14) a third shut-off means (16) is connected to said first incoming line segment (6), that to said first outgoing line segment (8) a fourth shut-off means (20) is connected, that the filling volume of said pump (14) is substantially greater than said filling volume from said port (2) to said connection point (21) with said first incoming line segment (6) and said second outgoing line segment (9), that the physiologically compatible aqueous solution is an infusion solution and that the control means (22) alternately switches said shut-off means (11, 15, 16, 20) and said pump (14) in a liquid infusion mode and the blood taking mode, respectively.

2. Method for operating the apparatus according to claim 1, characterized in that said control means (22) in the infusion mode switches said pump (14) into the expansion phase (filling stroke), opens said first shut-off means (11) and closes said second and third shut-off means (15 and 16) and switches in the discharge stroke said pump (14) into the compression phase, that it opens said first shut-off means (15) or said third and fourth shut-off means (16, 20) and that said control means (22) in the blood taking mode closes said first shut-off means, opens said second shut-off means (15), closes said third and fourth shut-off means (16, 20) and switches said pump (14) into the expansion phase and switches after having filled said pump (14) the shut-off means (15, 16, 20) and said pump (14) over into the compression phase.

3. Apparatus for taking of samples of blood and other body fluids comprising a port (2) to a body vessel (3) of which a first line (6) extends, said line having a sample measuring or respectively taking means (18) and a pump (14), further comprising a container (12) being connected to said first line (6) via a second line (10) and having a physiologically compatible aqueous solution, further comprising a first shut-off means (11) being disposed in said second line (10) and a control means (22) for activating said first shut-off means and said pump (14), characterized in that said pump (14) is connected with the end of said first line (6) being opposite said port (2) and has a filling volume which is substantially greater than the inner volume of said first line (6), that said first line (6) comprises a second shut-off means (15) within the area of said blood port (2), that the physiologically compatible aqueous solution is an infusion solution and that the control means (22) alternately switches said shut-off means (11, 15) in the liquid infusion mode and the blood taking mode, respectively.

4. Method for operating the apparatus according to claim 3, characterized in that said control means (22) in the infusion mode switches said pump (14) into the expansion phase (filling stroke), opens said first shut-off means (11) and closes said second shut-off means (15) and switches after the pump has been filled said pump (14) into the compression phase (discharge stroke), that it closes said first shut-off means (11) and opens said second shut-off means (15) and that said control means (22) in the blood taking mode closes said first shut-off means (11), opens said second shut-off means (15), switches said pump (14) into the expansion phase (filling stroke) whereby the taken-off blood is led through said sample taking means (18), subsequently said pump (14) switches into the compression phase (discharge stroke) and after discharge of said pump (14) switches back into the infusion mode.

5. Apparatus according to claim 1 or 3 characterized in that air detector (19) is provided in said line segments (6, 7, 8, 9).

6. Apparatus according to claim 1 or 3, characterized in that said pump (14) is a pneumatically operated pump which swings between a compression phase and an expansion phase.

7. Apparatus according to claim 6, characterized in that said pump (14) consists of a pump chamber (24), which is separated by a flexible membrane (25) into a first pump chamber portion (26) and a second pump chamber portion (27), and of a pneumatical drive member (28) for the pump chamber (24).

8. Apparatus according to one of claims 1, 3, 5, 6 or 7, characterized in that the sample taking means (18) is an electrochemical measuring unit or a filter unit for separating plasma.

## Revendications

1. Dispositif pour la prise d'essais de sang ou d'autres fluides corporels avec un raccordement (2) à un récipient corporel (3), d'où part un premier conduit (6 - 9) qui présente un dispositif (18) de mesure ou de prise d'essais et une pompe (14), un récipient (12) comprenant une solution aqueuse physiologiquement compatible, relié au premier conduit (6 - 9) par un deuxième conduit (10), un premier organe de blocage (11) placé dans le deuxième conduit (10) et un dispositif de commande (22) pour l'actionnement du premier organe de blocage (11) et de la pompe (14), caractérisé en ce que l'extrémité arrière du premier conduit (6 - 9) est ramenée vers l'extrémité avant du premier conduit en créant une boucle fermée, en ce que le dispositif de mesure ou de prise d'essais (18) est monté dans le premier conduit (6 - 9) avec une division du premier conduit en sections de conduit d'amenée (6, 7) et en sections de conduit de déviation (8, 9), en ce qu'un deuxième organe de blocage (15) est monté dans la première section de conduit (6) entre le point de liaison (21) avec la seconde section de dérivation (9) et le point de liaison avec le deuxième conduit (10), en ce qu'en aval du deuxième organe de blocage (15) la première section de conduit (6) d'amenée comprend la pompe (14), et en aval de la pompe (14) un troisième organe de blocage (16) est monté dans la première section de conduit d'amenée (6), en ce qu'un quatrième organe de blocage (20) est monté dans le premier conduit d'amenée (8), en ce que le volume plein de la pompe (14) est sensiblement plus important que le volume plein du raccordement (2) jusqu'au point de liaison (21) avec la première section de conduit d'amenée (6) et la deuxième section de conduit de dérivation (9), en ce que la solution aqueuse physiologiquement compatible est une solution de perfusion et en ce que le dispositif de commande (22) introduit tour à tour les organes de blocage (11, 15, 16, 20) et la pompe (14) dans le fonctionnement d'amenée de fluide et dans le fonctionnement de prélèvement de sang.

2. Procédé pour le fonctionnement du dispositif selon la revendication 1, caractérisé en ce que le dispositif de commande (22) dans le fonctionnement en perfusion enclenche la pompe (14) dans la phase d'expansion (phase de remplissage) ouvre le premier organe de blocage (11) et ferme le deuxième et le troisième organes de blocage (15 et 16) et enclenche dans la phase de vidage la pompe (14) dans la phase de compression, ouvre le premier organe de blocage (15) ou le troisième et le quatrième organes de blocage (16, 20) et en ce que le dispositif de commande (22) lors du fonctionnement en prise de sang ferme le premier organe de blocage (11), ouvre le deuxième organe de blocage (15), ferme les troisième et quatrième organes de blocage (16, 20) et enclenche la pompe (14) dans la phase d'expansion et commute dans la phase de compression après le remplissage de la pompe (14) les organes de blocage (15, 16, 20) et la pompe (14).

3. Dispositif de prélèvement de sang ou d'autres fluides corporels avec un raccordement (2) à un récipient corporel (3), d'où sort un premier conduit (6), qui présente un dispositif de mesure ou de prise d'essais (18) et une pompe (14), un réservoir (12) comprenant une solution aqueuse compatible physiologiquement et relié avec le premier conduit (6) par un deuxième conduit (10) un premier organe de blocage (11) monté dans le deuxième conduit (10) et un dispositif (22) pour l'actionnement du premier organe de blocage (11) et de la pompe (14), caractérisé en ce que la pompe (14) est reliée à l'extrémité du premier conduit (6) faisant face au raccordement (2) et présente un volume plein, qui est sensiblement plus important que le volume intérieur du premier conduit (6), en ce que le premier conduit (6) présente dans la zone du raccordement sanguin (2) un deuxième organe de blocage (15), en ce que la solution aqueuse physiologiquement compatible est une solution de perfusion et en ce que le dispositif de commande (22) enclenche tour à tour les organes de blocage (11, 15) dans le fonctionnement d'amenée de fluide et dans le fonctionnement d'enlèvement de sang.

4. Procédé de mise en oeuvre du dispositif selon la revendication 3, caractérisé en ce que le dispositif de commande (22) dans le fonctionnement de perfusion enclenche la pompe (14) dans la phase d'expansion (phase de remplissage), ouvre le premier organe de blocage (11) et ferme le deuxième organe de blocage (15) et après le remplissage enclenche la pompe (14) dans la phase de compression (phase de vidage) ferme le premier organe de blocage (11) et ouvre le deuxième organe de blocage (15) et en ce que le dispositif de commande (22) en fonctionnement en prise de sang ferme le premier organe de blocage (11) ouvre le deuxième organe de blocage (15), enclenche la pompe (14) dans la phase d'expansion (phase de remplissage), le sang prélevé étant amené par le dispositif de prélèvement de sang (18), enclenchant ensuite la pompe (14) dans la phase de compression (phase de vidage) et commutant à nouveau dans le fonctionnement à perfusion après vidage de la pompe (14).

5. Dispositif selon la revendication 1 ou 3 caractérisé en ce qu'un détecteur d'air (19) est disposé dans les sections de conduit (6, 7, 8, 9).

6. Dispositif selon la revendication 1 ou 3, caractérisé en que la pompe (14) est une pompe actionnée pneumatiquement, qui alterne entre une phase de compression et une phase d'expansion.

7. Dispositif selon la revendication 6, caractérisé en que la pompe (14) se compose d'une première chambre de pompe (24), qui est divisée par une membrane flexible (25) en une première partie de chambre de pompe (26) et une deuxième partie de chambre de pompe (27), et d'une pièce d'entraînement pneumatique (28) pour la chambre de pompe (24).

8. Dispositif selon l'une des revendications 1, 3, 5, 6 ou 7 caractérisé en ce que le dispositif de prélèvement (18) est un dispositif de mesure électrochimique ou unité de filtrage pour la séparation de plasma.

Fig.1

Fig.2